# EUROPEAN PATENT SPECIFICATION

(11) **EP 0 711 130 B1**
(45) Date of publication and mention of the grant of the patent: **06.11.2002**
(21) Application number: 94925128.4
(22) Date of filing: 28.07.1994
(51) Int. Cl.: A61B 18/00, A61M 25/01

(54) **CATHETER WITH BENDABLE TIP ASSEMBLY**
KATHETER MIT BIEGBAREM ENDSTÜCK
CATHETER AVEC EMBOUT FLEXIBLE

(30) Priority: 30.07.1993 US 99843; 30.07.1993 US 100739
(43) Date of publication of application: 15.05.1996
(73) Proprietor: Boston Scientific Limited, Saint Michael, Barbados, West Indies (BB)
(72) Inventor: HOUSER, Russell, A., Livermore, CA 94566 (US); BOURNE, Tom, Mountain View, CA 94040 (US)
(74) Representative: Adams, William Gordon
(86) International application number: US9408505
(87) International publication number: WO95003742

(56) References cited:
- EP-A- 0 600 676
- US-A- 3 605 725
- US-A- 4 873 983
- US-A- 4 998 933
- US-A- 5 040 543

## Description

This invention relates to bendable tip assemblies for catheters.

Cardiac mapping is used to locate aberrant electrical pathways and currents emanating within the heart. Such aberrant pathways cause irregular contractions of the heart muscle resulting in life-threatening patterns of disrhythmias.

Intercardiac mapping requires careful positioning of the electrodes within the heart. Various steering mechanisms for catheters carrying such electrodes have heretofore been developed and used.

To provide catheters having different curve configurations to access various endocardial sites, physicians have to stock and use a number of different catheters, each of which provides a different curve configuration. Commercially available catheters, thus, come in sets, which often provide four to seven different curve configurations by using different catheters.

This approach presents serious disadvantages, because the physician often must repeatedly remove and re-insert these different catheters to complete a procedure on a given patient.

US-A-3, 605, 725 discloses a known controlled wire catheter having features included in the preamble of claim 1.

According to the present invention, there is provided a catheter including an elongate body defining a distal end, a proximal end and a longitudinally extending bore, and having a tip assembly associated with the distal end of the elongate body, the tip assembly defining a distal end and a proximal end and being adapted to bend relatively to the elongate body, and there being a steering wire operably connected to the tip assembly and adapted to apply a bending force to the tip assembly; characterised by a rotatable stiffening member including four stiffening parts of differing lengths.

The present catheter is useable in both diagnostic and therapeutic applications and enables a physician to swiftly and accurately change the configuration or steering characteristics of the distal curve of the catheter as it is steered within the body of a patient. The catheter allows physicians to access many more endocardium sites than heretobefore achievable by a single catheter. The catheter enables a physician to alter the physical characteristics of it when inserted within a living body by manipulation of external controls.

A longer fulcrum-to-distal tip distance can be provided for accessing and measuring electrical activity in portions of the heart such as the coronary sinus, the right ventricular overflow tract and the HIS bundle. Similarly, a shorter fulcrum-to-tip distance can be used to access such areas as the high right atrium and the right and left ventricle apex of the heart.

For a better understanding of the invention and to show how the same may be carried into effect, reference will now be made, by way of example, to the accompanying drawings, in which:-
Figure 1 is a view of the catheter with a tip portion broken away and shown in cross-section on a greatly enlarged scale and showing a rotatable sleeve;
Figure 2 is a perspective view showing the configuration of the distal end of the rotatable sleeve shown in Figure 1;
Figure 3 is a fragmentary cross-sectional view showing the control mechanism in the distal portion of the handle of the catheter; and
Figure 4 is a top sectional view of the handle of the device on a greatly enlarged scale.

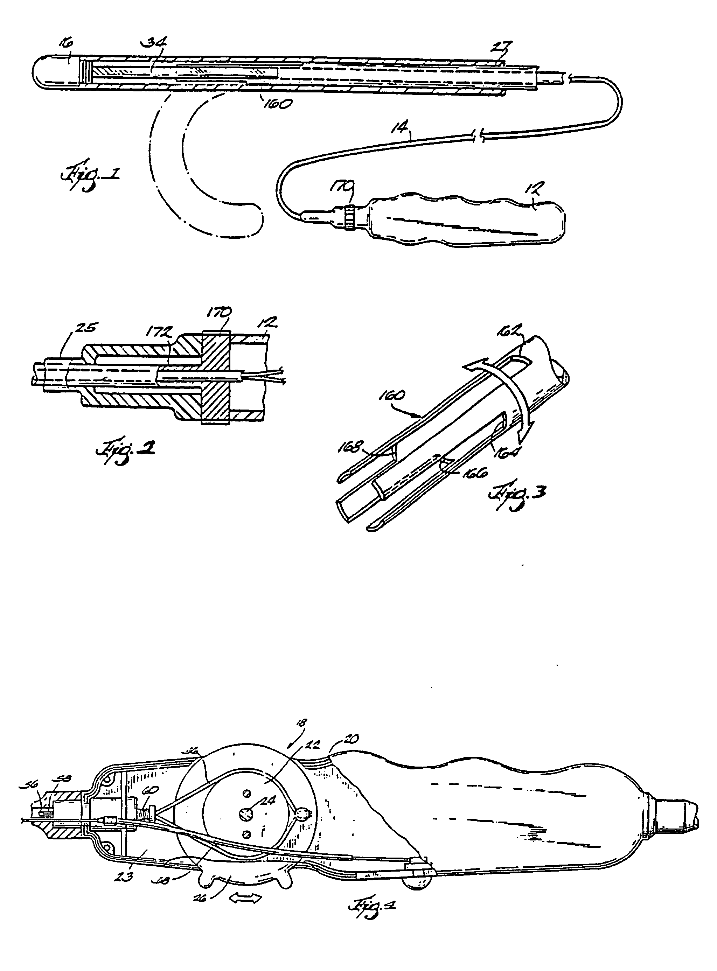

Figure 1 shows a steerable catheter that includes a handle 12, a catheter tube or body 14 and a steerable distal tip assembly 16. In use, the catheter provides electrophysiology diagnosis or therapy in the interior regions of the heart.

When used for this purpose, a physician grips the handle 12 and manoeuvres the catheter body 14 through a main vein or artery (which is typically the femoral vein) into the interior region of the heart that is to be treated. The physician then further steers the distal tip assembly 16 to place it in contact with the tissue that is to ablated. The physician directs energy to an electrode in the assembly 16 to ablate the tissue contacted.

As Figure 4 shows, the handle 12 encloses a steering mechanism which may be in the form of a rotating cam wheel of the type shown in U.S. Patent 5,195,968. While one form of steering mechanism 18 is shown for purposes of illustration, it will be understood that many other mechanisms that allow for selective pulling of the steering wires in the catheter can be substituted.

The handle assembly 12 includes a housing 20 that encloses the steering mechanism 18. The steering mechanism 18 includes a rotatable wheel or cam 22 carried on a shaft 24 within the housing 20. The rotatable cam 22 and control knob 26 are attached to shaft 24 by splines. Clockwise movement of the control knob 26 rotates the cam 22 clockwise pulling on wire 56. Counter-clockwise movement of the control knob 26 reverses the direction of each of these movements and results in pulling wire 58. Various other mechanisms can be substituted to apply tension to wires 56 and 58 in place of that shown in Figure 4.

The steering wires 56 and 58 exit the front of the housing 20 through the interior bore of a tension screw assembly 60. The distal ends of the steering wires 56 and 58 are attached to a steering wire or spring in the electrode tip assembly 16.

The catheter body 14 is a flexible shaft attached to the handle 12. While it can be variously constructed, in a preferred embodiment, the catheter body 14 is a length of stainless steel coiled into a flexible spring 25 enclosing an interior bore 27 which in turn is enclosed in a braided sheath 29 of plastic material. The steering wires 56 and 58 preferably pass through the interior bore 27, which leads to the distal tip assembly 16 where the steering wires are attached to a bendable main support wire 34. In the illustrated embodiment, the main support wire 34 is made of stainless steel flat wire stock in an elongated shape about 0.889mm (0.035 inch) wide and about 0.127mm (0.005 inch) thick. The main support wire 34 is about 76.2mm (3 inches) in total length.

Reverting to Figures 1 to 3, a tube 160 is rotatable relative to the steering wire 34.

A rotatable sleeve concentric with the spring 25 and located within bore 27 is attached to the tube 160 for the purpose of applying rotational forces thereto. The tube 160 is provided with a distal end having circumferential segments 162, 164, 166 and 168 of differing lengths. Each of these circumferential segments provides a fulcrum or stiffening member spaced a different distance from the distal tip of the catheter and thus provides for as many different curvature shapes of the tip as there are segments.

The tube 160 is rotated by applying a force to a control knob 170 located in the handle 12. The tube 160 is connected to the control knob 170 by means of a torque transmitting tube 172. Since the control wire 34 will tend to bend the distal tip 16 from side to side in a single plane, the fulcrum length provided by rotatable tube 160 is dependent on which of the segments 162, 164, 166 or 168 is positioned in the plane in which the control wire 34 bends. Appropriate markings on the handle 12 can be provided to indicate the position in which the control knob 170 is rotated. Thus, the physician can readily change the fulcrum length or stiffness characteristics of the distal tip of the catheter by manually rotating the knob 170.

## Claims

1. A catheter including an elongate body (14)defining a distal end, a proximal end and a longitudinally extending bore, and having a tip assembly (16) associated with the distal end of the elongate body, the tip assembly defining a distal end and a proximal end and being adapted to bend relatively to the elongate body, and there being a steering wire (56) operably connected to the tip assembly and adapted to apply a bending force to the tip assembly; **characterised by** a rotatable stiffening member (160) including four stiffening parts (162, 164, 166, 168) of differing lengths.

2. A catheter as claimed in claim 1, further comprising a handle (12) associated with the proximal end of the elongate body (14) and a stiffening member control device (170) located on the handle.

3. A catheter as claimed in claim 2, further comprising a torque transmitting tube (172) connecting the stiffening member control device (170) to the rotatable stiffening member (160).

4. A catheter as claimed in any one of the preceding claims, wherein the tip assembly (16) includes at least one electrode.

## Patentansprüche

1. Katheter mit einem langgestreckten Körper (14), der ein distales Ende, ein proximales Ende und eine sich längs erstreckende Bohrung definiert und ein Endstück (16) hat, das dem distalen Ende des langgestreckten Körpers zugeordnet ist, wobei das Endstück ein distales Ende und ein proximales Ende definiert und angepaßt ist, sich relativ zu dem langgestreckten Körper zu biegen, wobei ein Steuerdraht (56) vorgesehen ist, der mit dem Endstück wirkverbunden ist und angepaßt ist, eine Biegekraft auf das Endstück aufzubringen; **gekennzeichnet durch** ein drehbares Aussteifungsglied (160), das vier Aussteifungsteile (162, 164, 166, 168) von verschiedener Länge aufweist.

2. Katheter nach Anspruch 1, ferner umfassend eine Handhabe (12), die dem proximalen Ende des langgestreckten Körpers (14) zugeordnet ist, und eine Aussteifungsglied-Steuereinrichtung (170), die an der Handhabe angeordnet ist.

3. Katheter nach Anspruch 2, ferner umfassend eine drehmomentübertragende Röhre (172), welche die Aussteifungsglied-Steuereinrichtung (170) mit dem drehbaren Aussteifungsglied (160) verbindet.

4. Katheter nach einem der vorhergehenden Ansprüche, wobei das Endstück (16) wenigstens eine Elektrode aufweist.

## Revendications

1. Cathéter comprenant un corps allongé (14) définissant une extrémité distale, une extrémité proximale et un conduit s'étendant longitudinalement, et comportant un ensemble de pointe (16) associé à l'extrémité distale du corps allongé, l'ensemble de pointe définissant une extrémité distale et une extrémité proximale et étant adapté pour s'incurver par rapport au corps allongé, et un fil de guidage (56) étant présent, connecté de façon opérationnelle à l'ensemble de pointe, et adapté pour appliquer une force de flexion à l'ensemble de pointe ; **caractérisé par** un élément de raidissement rotatif (160) comprenant quatre parties de raidissement (162, 164, 166, 168) de longueurs différentes.

2. Cathéter selon la revendication 1, comprenant de plus une poignée (12) associée à l'extrémité proximale du corps allongé (14) et un dispositif de commande d'élément de raidissement (170) disposé sur la poignée.

3. Cathéter selon la revendication 2; comprenant de plus un tube de transmission de couple (172) reliant le dispositif de commande d'élément de raidissement (170) à l'élément de raidissement rotatif (160).

4. Cathéter selon l'une quelconque des revendications précédentes, dans lequel l'ensemble de pointe (16) comprend au moins une électrode.
